# EUROPEAN PATENT SPECIFICATION

(11) **EP 3 000 435 B1**
(45) Date of publication and mention of the grant of the patent: **13.09.2017**
(21) Application number: 15185450.2
(22) Date of filing: 20.05.2005
(51) Int. Cl.: A61F 2/16

(54) **INTRAOCULAR LENS**
INTRAOKULARE LINSE
LENTILLE INTRAOCULAIRE

(43) Date of publication of application: 30.03.2016
(62) Divisional of application: 05744110.7
(73) Proprietor: Kowa Company, Ltd., Nagoya-shi, Aichi 460-0003 (JP)
(72) Inventor: MARUNAKA, Akinori, Kasugai-shi, Aichi 487-0032 (JP); TANAKA, Masayoshi, Kakamigahara-shi, Gifu 509-0108 (JP)
(74) Representative: Serjeants LLP

(56) References cited:
- EP-A- 1 358 858
- EP-A2- 0 438 043
- US-A- 4 808 181
- US-A- 4 961 746
- US-A- 5 074 942

## Description

### TECHNICAL FIELD

The present invention relates to an intraocular lens of one-piece design in which the optical portion and the supporting portions are integrally formed of soft material; and relates in particular to an intraocular lens able to be deformed by means of folding or rolling into a more compact shape for insertion into the eye.

### BACKGROUND ART

One well-known method used in the past for treating the ocular disorder known as a cataract involves removing the lens of the eye, and employing an intraocular lens as a substitute for the lens. An intraocular lens of this kind is used by being inserted into the capsule from which the lens has been removed; the lens has an optical portion, which functions in place of the lens of the eye, and a support portion for positioning and immobilizing the optical portion within the capsule.

In cataract treatment employing an intraocular lens of this kind, an incision is made in part of the patient's eye, the lens of the eye is withdrawn through the incision, and then the intraocular lens is inserted into the capsule. In order to reduce demands on patient by the surgery and to avoid subsequent complications, the incision wound made during surgery should be small. However, even if the incision made during surgery is small, if the intraocular lens being inserted is large in size relative to the size of the incision, the incision which has deliberately been made small will inevitably become enlarged during insertion.

For this reason, in recent years, intraocular lenses of foldable type formed of materials having resilience and softness so as to be readily inserted through a small incision have been used to good advantage. Such intraocular lenses are folded or rolled at the time of insertion for insertion into the capsule through an incision, whereupon the intraocular lens now situated within the capsule recovers its initial shape owing to its resilience, whereby the supporting portions deploy to securely position the lens.

More recently, with intraocular lenses of foldable type, it has become common to employ a specific insertion instrument in order to insert the lens into the eye. Where such an insertion instrument is employed for insertion, the intraocular lens of foldable type will be positioned on the insertion instrument, with its pair of supporting portions oriented in the longitudinal direction for example; and will then be folded or rolled about an axis extending in the longitudinal direction to make it compact in the width direction. The intraocular lens positioned thusly will then be inserted into the eye by pushing the posterior end of the optical portion with a plunger; in many instances, in order to avoid damage to the thin, elongated supporting portions during the process, the support portion at the posterior end will initially be folded and superposed onto the optical portion. Moreover, when the posterior end of the optical portion is pushed from the rear by the plunger, the support portion at the anterior end tends to become bent rearward due inter alia to friction against the peripheral wall of the insertion instrument, so in many instances the support portion at the anterior end will be superposed onto the optical portion as well. Thus, the supporting portions will typically be superposed onto the optical portion in this way during insertion of the intraocular lens into the capsule.

The use of silicone based polymers and the like as materials for intraocular lens is currently under study, but intraocular lenses of foldable type that use acrylic based polymers, which have a number of advantages particularly in terms of biocompatibility, optical properties, or fewer subsequent complications, are under study as well.

However, in many instances intraocular lenses employing such acrylic based polymers polymerized from acrylic acid esters or methacrylic acid esters as some of the polymer components tend to have high levels of viscosity as compared to silicone based polymers. Thus, when the intraocular lens is folded or rolled (this includes curling, rolling, etc.) during the process of inserting the intraocular lens into the capsule, there is a risk that superposed surfaces of the optical portion and the supporting portions will adhere together so that [the lens] does recover its initial shape despite the resilience of the optical portion and the supporting portions. In particular, since the supporting portions are of thin, elongated rod form, where they are made soft enough to permit folding or rolling it will be difficult to also endow them with recovery force sufficient to overcome adhesion. On the other hand, while it would be conceivable to make the supporting portions larger so as to easily overcome adhesion, making the supporting portions larger will not only make folding or rolling more difficult, but also poses the problem of a larger burden on the patient, due to enlargement of the incision wound during insertion into the capsule.

In order to address such problems, there has been proposed, for example in Patent Document 1 (JP-A-11-70130), an intraocular lens having an optical portion divided into center and outer peripheral portions, whose center portion constitutes a viscous portion and whose outer peripheral portion constitutes a non-viscous portion. With this intraocular lens, adhesion due to juxtaposition can be avoided, at least in the non-viscous portion.

With an intraocular lens such as that disclosed in Patent Document 1, the need to form a discrete viscous portion and non-viscous portion makes it difficult to effectively ensure high productivity, which is one advantage afforded by intraocular lenses of one-piece design. Furthermore, since the center portion of the optical portion is viscous, the supporting portions superposed thereon will tend to stick, and thus in consideration of circumstances on the spot there is unlikely to be notable effect in avoiding adhesion of the supporting portions to the optical portion.

Patent Document 1: Japanese Unexamined Patent Application 11-70130
The preamble of claim 1 is derivable from EP 1358858 A1.

### DISCLOSURE OF THE INVENTION

### Problem the Invention Attempts to Solve

With the foregoing in view, it is an object of the present invention to provide a one-piece intraocular lens of novel structure that effectively avoids adhesion of the supporting portions to the optical portion in the intraocular lens, thereby advantageously achieving an intraocular lens that is fabricated from an acrylic polymer or other material with high adhesion force, and that is nevertheless endowed with good workability and so on.

### Means for Solving the Problem

The present invention provides an intraocular lens of one-piece type comprising:
an optical portion of generally circular shape in front view and including a lens zone having prescribed optical characteristics; and
supporting portions that extend radially outwardly from the optical portion, and that with the lens inserted in an eye, are configured to be disposed in contact against an inside surface of an outer circumferential part of a capsule thereby holding the optical portion positioned within the capsule, each supporting portion having a projecting distal end side and a basal end side, the optical portion and supporting portions are integrally formed with a foldable or rollable soft material, wherein
a viscous material is employed as the soft material,
the supporting portions have rough surface portions with reduced viscosity at least at portions that are adapted to be superposed on the optical portion when the lens is folded or rolled and inserted in to the eye,
   the supporting portions have a reduced transparency by means of the rough surface portions in comparison with the optical portion, and
   the rough surface portions are formed at least on the projecting distal end sides and basal end sides of the supporting portions.

In the intraocular lens of structure according to the present invention, the viscosity reducing portions are constituted by subjecting the supporting portions to a surface roughening process, whereby it is possible to advantageously avoid increased radial thickness of the supporting portion in the direction of the optical axis, in association with disposing the viscosity reducing portions on the supporting portions.

Moreover, by subjecting the supporting portions to a surface roughening process, sufficient visibility can be assured even where insertion takes place within a liquid, thereby facilitating the procedure for inserting the intraocular lens into the eye and reducing the burden on the technician.

Specifically, one problem encountered in the past is that the operation of positioning the intraocular lens within the eye is carried out substantially within a liquid such as the aqueous humor which fills the eye is difficult owing to the extremely low visibility of an intraocular lens that is clear or slightly yellow-colored, thus increasing the burden on the technician. One method proposed for solving this problem in the case of three-piece lenses is to color the supporting portions to a blue color which is highly visible in liquid; however, in the case of an intraocular lens of one-piece design, since the optical portion and the supporting portions are integrally formed, the lens is substantially clear in its entirety, which made it difficult to improve the visibility. While the use of different materials for the supporting portions and the optical portion could be contemplated, the use of separate materials poses the risk that the advantages of one-piece type intraocular lenses in terms of high productivity and so on will be lost; whereas if it is attempted to ensure visibility of the supporting portions while using the same material for the supporting portions and the optical portion, there is the risk of unavoidable limitations as to materials, and improved visibility of materials has proven difficult. In the present invention, however, by means of establishing appropriate morphology for the supporting portions, the transparence of the supporting portions can be reduced and their visibility in liquid improved, while at the same time effectively attaining the effect of reduced adhesion of the supporting portions to the optical portion. By so doing, the intraocular lens can be manipulated easily even after insertion into the eye, making it possible to smoothly carry out operations such as positioning of the lens. For this reason, the burden on the technician can be reduced, and the surgery can be made safer due to the simpler procedure, thus reducing risk to the patient.

Furthermore, by subjecting the supporting portions to a surface roughening process, it is possible to effectively reduce or avoid the occurrence of glare in the supporting portions. Specifically, during wear of the intraocular lens, there are instances in which the details of a subject being gazed upon can become indistinct due vision-impairing glare such as that caused by scattering of incident light at the outer periphery of the optical portion. Such glare is thought to occur with scattering of light incident on the supporting portions as well, and to be a possible factor in unclear vision, particularly where the pupils have become dilated in the dark. In the intraocular lens according to the present invention, transmission of light is prevented by means of subjecting the supporting portions to a surface roughening process, thereby preventing such glare. In particular, by subjecting the supporting portion to a surface roughening process in a portion thereof inclusive of the basal end situated closer in proximity to the optical portion, it is possible to more effectively prevent the occurrence of glare.

As will be apparent from the preceding description, in the intraocular lens of structure according to the present invention, by means of the viscosity reducing portions formed on the supporting portions it is possible to prevent strong adhesion between the superposed faces of the optical portion and the supporting portions in association with folding or rolling of the intraocular lens for insertion into the capsule. Therefore, the supporting portions can easily recover their original shape after insertion into the capsule, and can easily and stably position and support the optical portion at the prescribed location within the capsule.

### BRIEF DESCRIPTION OF THE DRAWINGS

[FIG. 1] FIG. 1 is a front elevational view of an intraocular lens.
[FIG. 2] FIG. 2 is a rear elevational view of the intraocular lens of FIG. 1.
[FIG. 3] FIG. 3 is a side elevational view of the intraocular lens of FIG. 1.
[FIG. 4] FIG. 4 is a side elevational view of a principal part of an intraocular lens.
[FIG. 5] FIG. 5 is a rear elevational view of the intraocular lens of FIG. 4.
[FIG. 6] FIG. 6 is a side elevational view of a principal part of an intraocular lens.
[FIG. 7] FIG. 7 is a rear elevational view of the intraocular lens of FIG. 6.
[FIG. 8] FIG. 8 is a rear elevational view of an intraocular lens according to an embodiment in accordance with the present invention.
[FIG. 9] FIG. 9 is a side elevational view of a principal part of an intraocular lens.
[FIG. 10] FIG. 10 is a rear elevational view of a principal part of the intraocular lens of FIG. 9.
[FIG. 11] FIG. 11 is a side elevational view of a principal part of an intraocular lens.
[FIG. 12] FIG. 12 is a rear elevational view of a principal part of the intraocular lens of FIG. 11.
[FIG. 13] FIG. 13 is a side elevational view of a principal part of an intraocular lens.
[FIG. 14] FIG. 14 is a rear elevational view of a principal part of the intraocular lens of FIG. 13.
[FIG. 15] FIG. 15 is a side elevational view of a principal part of an intraocular lens.
[FIG. 16] FIG. 16 is a rear elevational view of a principal part of the intraocular lens of FIG. 15.
[FIG. 17] FIG. 17 is a side elevational view of a principal part of an intraocular lens.
[FIG. 18] FIG. 18 is a rear elevational view of a principal part of the intraocular lens of FIG. 17.
[FIG. 19] FIG. 19 is a side elevational view of a principal part of an intraocular lens.
[FIG. 20] FIG. 20 is a rear elevational view of a principal part of the intraocular lens of FIG. 19.
[FIG. 21] FIG. 21 is a side elevational view of a principal part of an intraocular lens.
[FIG. 22] FIG. 22 is a rear elevational view of a principal part of the intraocular lens of FIG. 21.
[FIG. 23] FIG. 23 is a side elevational view of a principal part of an intraocular lens.
[FIG. 24] FIG. 24 is a rear elevational view of a principal part of the intraocular lens of FIG. 23.
[FIG. 25] FIG. 25 is a side elevational view of a principal part of an intraocular lens according to an embodiment in accordance with the present invention.
[FIG. 26] FIG. 26 is a side elevational view of a principal part of an intraocular lens.
[FIG. 27] FIG. 27 is a rear elevational view of a principal part of the intraocular lens of FIG. 26.
[FIG. 28] FIG. 28 is a side elevational view of a principal part of an intraocular lens.
[FIG. 29] FIG. 29 is a rear elevational view of a principal part of the intraocular lens of FIG. 28.
[FIG. 30] FIG. 30 is a side elevational view of a principal part of an intraocular lens.
[FIG. 31] FIG. 31 is a rear elevational view of a principal part of the intraocular lens of FIG. 30.
[FIG. 32] FIG. 32 is a side elevational view of a principal part of an intraocular lens.
[FIG. 33] FIG. 33 is a rear elevational view of a principal part of the intraocular lens of FIG. 32.
[FIG. 34] FIG. 34 is a side elevational view of a principal part of an intraocular lens.
[FIG. 35] FIG. 35 is a rear elevational view of a principal part of the intraocular lens of FIG. 34.
[FIG. 36] FIG. 36 is a rear elevational view of an intraocular lens.
[FIG. 37] FIG. 37 is a rear elevational view of an intraocular lens.
[FIG. 38] FIG. 38 is a rear elevational view of an intraocular lens.
[FIG. 39] FIG. 39 is a rear elevational view of an intraocular lens.

### EXPLANATION OF NUMERALS

- 10: Intraocular lens
- 12: Optical portion
- 16: Supporting portions
- 26: Supporting portion anterior surface
- 28: Supporting portion posterior surface
- 32: Projecting part
- 34: Rib part
- 36: Recess
- 38: Rough surface portion

### BEST MODE FOR CARRYING OUT THE INVENTION

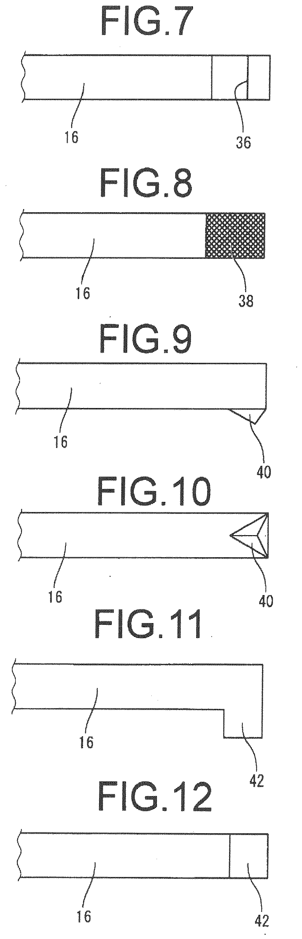

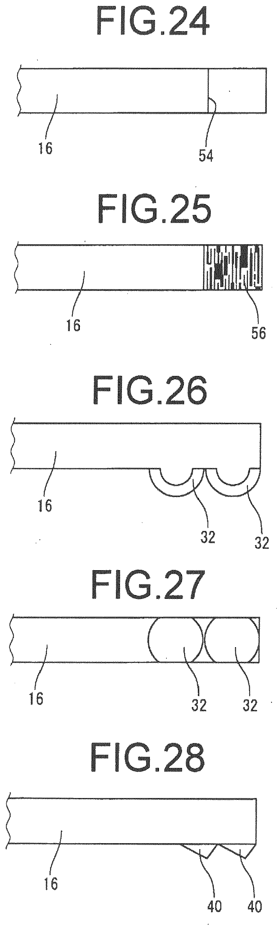

A fuller understanding of the present invention will be provided through the following detailed description of the embodiments, with reference to the accompanying drawings. Only the passages of the description described with reference to FIGS. 8 and 25 respectively, fall within the scope of the present invention as defined in the appended claims. The other parts of the description and figures are provided by way of example only, for a better understanding of the invention. Furthermore, only embodiments having rough surface portions formed at least on projecting distal end sides and basal end sides of the supporting portions fall within the scope of the present invention as defined in the appended claims.

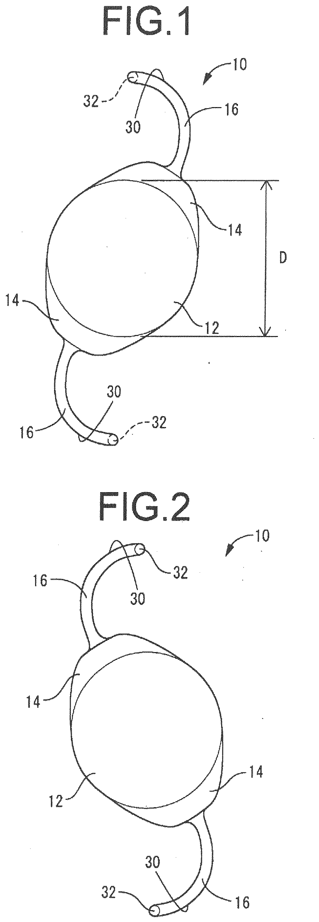

First, FIGS. 1 through 3 depict an intraocular lens 10 of foldable type. This intraocular lens 10 includes an optical portion 12, a pair of coupling portions 14, 14, and a pair of supporting portions 16, 16.

This intraocular lens 10 having the optical portion 12, the coupling portions 14, 14, and the supporting portions 16, 16 may be formed of any of various materials endowed with visible light transmissivity sufficient to give an intraocular lens of foldable type, and endowed with excellent softness and certain amount of elasticity. In preferred practice, the soft material will have glass transition temperature of 30°C or higher and refractive index of 1.51 or lower. Such soft materials enable the intraocular lens 10 to be easily folded or rolled up at normal temperature, so as to make it more compact as well as further facilitating insertion into the eye during the implantation process. In particular, an acrylic polymer having viscosity at normal temperature is favorably employed.

Specifically, the materials taught in JP-A- 10-24097 and JP Patent No. 3494946 are suitable for use as materials for forming the intraocular lens 10. Of these, monomers including one or more (meth)acrylic acid esters such as those listed in (i) below are preferred as they endow the intraocular lens with exceptional shape recovery. Optional suitable monomers such as those listed in (ii) below may be included also. Also, additives such as those listed in (iii) below may be added as needed.

### (i) Included monomers

Linear, branched, or cyclic alkyl (meth)acrylates such as the following:
methyl (meth)acrylate, ethyl (meth)acrylate, butyl (meth)acrylate, cyclohexyl (meth)acrylate, etc.
Hydroxyl group-containing (meth)acrylates such as the following:
   hydroxyethyl (meth)acrylate, hydroxybutyl (meth)acrylate, diethylene glycol mono(meth)acrylate, etc.
Aromatic ring-containing (meth)acrylates such as the following:
   phenoxyethyl (meth)acrylate, phenyl (meth)acrylate, phenyl ethyl (meth)acrylate, etc.
Fluorine-containing (meth)acrylates such as the following:
   trifluoroethyl (meth)acrylate, tetrafluoropropyl (meth)acrylate, etc.
Silicone-containing (meth)acrylates such as the following:
   trimethylsiloxy dimethylsilylmethyl (meth)acrylate, trimethylsiloxy dimethylsilylpropyl (meth)acrylate, etc.

Herein, the expression "(meth)acrylates" is used to refer collectively to "acrylates" and "methacrylates"; this convention will also be employed for other (meth)acrylic derivatives to be discussed later.

### (ii) Optional monomers

(Meth)acrylamide and derivatives thereof such as the following:
(meth)acrylamide, N,N-dimethyl (meth)acrylamide, etc.
N-vinyl lactams such as the following:
   N-vinyl pyrrolidone, etc.
   styrene or derivatives
crosslinking monomers such as the following:
   butanediol di(meth)acrylate, ethylene glycol di(meth)acrylate

### (iii) Additives

thermal polymerization initiators, photopolymerization initiators, photosensitizers, etc.
dyes etc.
UV absorbers etc.

During the process of integrally molding the illustrated intraocular lens 10 from these monomer materials, it is possible to use any of the various methods known in the conventional art. For example, the intended intraocular lens 10 could be obtained by a forming process involving cutting, or by a forming process involving molding. Where a cutting process is employed, prescribed polymer components selected from monomer materials such as the above will undergo polymerization to form a lens blank of appropriate shape such as a rod, block, or slab. The lens blank will then undergo a cutting process using a lathe or the like to produce the intraocular lens 10 of the desired shape. Where a molding process is employed, using a mold cavity that corresponds in shape to the intended shape of the intraocular lens 10, prescribed polymer components selected from monomer materials such as the above will be introduced into the mold cavity and subjected in situ to an appropriate polymerization procedure to obtain the intraocular lens 10 of the desired shape. With the intraocular lens 10 since the acrylic polymer exhibiting viscosity at normal temperature is selected as the material, the aforesaid molding process is favorably employed in order to ensure its production efficiency. The method of polymerization of the monomer material will be selected appropriately according to the particular monomer material, from among the various known methods such as thermal polymerization, photopolymerization, or some combination of these.

In the integrally formed intraocular lens 10 described above, the optical portion 12 is of generally disk shape which is circular in front view. The center portion of the optical portion 12 or the entire zone thereof constitutes a lens zone having optical properties that allow it to function as a substitute for the lens of the human eye. The lens surfaces of the optical portion 12 (an optical portion anterior surface 18 and an optical portion posterior surface 20) may take any of various shapes according to the required optical properties. The two surfaces 18, 20 are established by appropriate combination of concave surfaces, convex surfaces, planar surfaces, and so on. In this example, there is employed an optical portion 12 of convex lens shape, in which the optical portion anterior surface 18 and the optical portion posterior surface 20 are both spherical convex surfaces. In the intraocular lens 10 according to this example, an anterior surface should be interpreted to a face located on a cornea side when the intraocular lens is inserted into a capsule, and an posterior surface should be interpreted to a face located on a retina side within the capsule.

Here, the diameter dimension: D of the optical portion 12 is preferably within a range of 4.5 - 7.5 mm. The radial thickness dimension (thickness): d of the optical portion 12 in the direction of the optical axis preferably has a maximum value of from 0.10 to 2.00 mm in view of operability like folding or rolling as well as optical characteristics.

A pair of coupling portions 14, 14 which extend a prescribed length in the circumferential direction are formed at the outer fringe part of the optical portion 12, projecting so as to be positioned at opposing locations along a direction lying across the diameter of the optical portion 12. Each coupling portion 14 is of generally trapezoidal shape in front view, gradually constricting in width in the circumferential direction on the projecting distal edge side.

The coupling portion 14 is composed of coupling portion anterior surfaces 22 constituted by sloping surfaces situated on one face in the direction of the optical axis (the optical portion anterior surface 18 side) and which, moving towards the outer fringe side, slope gradually towards the anterior surface side in the direction of the optical axis. Coupling portion posterior surfaces 24 situated on the other face are constituted by flat surfaces which extend perpendicular to the optical axis, so that moving towards the outer fringe side the coupling portion 14 as a whole increases gradually in thickness.

Furthermore, the radial thickness dimension (thickness) at the inner fringe part of the coupling portion 14 in the direction of the optical axis is approximately the same as the radial thickness dimension (thickness) at the outer fringe part of the optical portion 12 in the direction of the optical axis. The outer circumferential surface of the coupling portion 14 is curved in arc shape so as to conform to the respective outside contour of the optical portion 12, and smoothly connected to the outer circumferential surface of the optical portion 12.

The pair of supporting portions 16, 16, meanwhile, are integrally formed on each arcuate outer circumferential surface of the coupling portions 14, 14. The pair of supporting portions 16, 16 have thin, elongated rod shape, and are respectively projected outwardly in the diametrical direction of the optical portion 12 from the circumferential central portions of the coupling portions 14, 14, as well as extending in bowed configuration along the circumferential direction of the optical portion 12. When the intraocular lens 10 is inserted into the capsule, surfaces of the supporting portions 16, 16, which are located on the cornea side are referred to as supporting portion anterior surfaces 26, while the other surfaces of the supporting portions 16, 16, which are located on the retina side are referred to as supporting portion posterior surfaces 28. The outer circumferential side faces of the bowed portions of the pair of supporting portions 16, 16 constitute contact surfaces 30, 30 for disposition in contact against the inside surface of the capsule in the eye with the intraocular lens 10 inserted into the capsule. By means of this design, in the present embodiment, the optical portion 12 is supported positioned at the prescribed location within the capsule by means of the pair of supporting portions 16, 16.

According to this example, the supporting portions 16, 16 have viscosity reducing portions in the form of projecting parts 32, 32 formed on distal end portions thereof. Each projecting part 32 is formed so as to project out from the corresponding supporting portion posterior surface 28, with a semispherical shape.

The radial thickness dimension (thickness) of the supporting portions 16, 16 is smaller than the radial thickness dimension of the coupling portions 14, 14 at their outer circumferential edge. In this example, radial thickness is also slightly greater than radial thickness in the outer fringe part of the optical portion 12. The supporting portions 16, 16 extend from the edge of the outer circumferential end face of the coupling portions 14, 14, on the coupling portion anterior surfaces 22 thereof. That is, within a plane extending perpendicular to the optical axis, a pair of supporting portion anterior surfaces 26, 26 which constitute the surfaces on a first side of the supporting portions 16, 16 in the direction of the optical axis are positioned generally co-planar with the outer circumferential edge of the pair of coupling portion anterior surfaces 22; while the pair of supporting portion posterior surfaces 28, 28 situated on the other side of the supporting portions 16, 16 in the direction of the optical axis are positioned biased further towards the anterior surface side than are the pair of coupling portion posterior surfaces 24, 24.

The intraocular lens 10 having the structure discussed above, in its entirety including the optical portion 12 and the supporting portions 16, 16, can be folded or rolled in the appropriate direction to reduce its overall size. Then, following known procedures, an incision is made in part of the eye, the lens is removed from the site using suction etc., and the intraocular lens 10 reduced to small size is inserted through the incision wound and into the capsule. An appropriate insertion instrument (e.g., instruments disclosed in JP-A-2-156943 and JP-A-9-508810) may be used for the insertion operation if needed.

Where the intraocular lens 10 is inserted into the eye with this kind of insertion instrument, the intraocular lens 10 is oriented with the pair of supporting portions 16, 16 positioned at front and back in the insertion direction and the intraocular lens is folded or rolled about an axis extending in the direction of opposition of the supporting portions 16, 16, thereby advantageously reducing the intraocular lens 10 to small size in the direction generally orthogonal to the direction of opposition of the supporting portions 16, 16. The elongated intraocular lens 10 which has been folded or rolled compactly in this manner is positioned in the insertion instrument so that the insertion direction is coincident with the direction of opposition of the supporting portions 16, 16, i.e. the lengthwise direction. The intraocular lens 10 positioned thusly in the insertion instrument is then pushed from its posterior end in the insertion direction by the plunger, pushing it into the eye.

There is a risk that, if the plunger pushes the intraocular lens 10 from the rear with the pair of supporting portions 16, 16 left projecting out from the outer fringe part of the optical portion 12 towards either side in the insertion direction, the supporting portion 16 situated at the trailing end in the insertion direction will become damaged by the pushing force of the plunger; for this reason, in most cases the supporting portion 16 situated at the trailing end in the insertion direction will be folded in advance and the intraocular lens inserted into the eye with the trailing supporting portion 16 superposed against the optical surface of the optical portion 12. During insertion, the intraocular lens 10 will be inserted into the eye by means of the plunger pushing the outer fringe part of the optical portion 12 situated at the trailing end in the insertion direction.

As the intraocular lens is pushed along by the plunger, the supporting portion 16 situated at the leading end in the insertion direction is easily bent rearward due to friction with the peripheral wall of the insertion instrument, whereby the folded supporting portion 16 becomes superposed against the optical surface of the optical portion 12. The intraocular lens 10 of the present embodiment is thereby inserted into the eye, with the pair of supporting portions 16, 16 projecting out to either side in the insertion direction both superposed against the optical surface of the optical portion 12.

Where the intraocular lens 10 material includes an acrylic polymer exhibiting viscosity at normal temperature, in some instances the optical portion 12 and the supporting portions 16, 16 may tend to adhere together at their portions which are superposed during folding or rolling. Particularly with supporting portions of conventional design, since the supporting portions are superposed against the optical portion by being folded in the manner mentioned above during insertion of the intraocular lens, they tended to adhere to the optical surface of the optical portion, and where the supporting portions have narrow elongated rod shape, it was difficult to impart to them resilient force sufficient to overcome the adhered state, with the risk that the intraocular lens will not readily recover its initial shape naturally through resilient force after being inserted into the eye.

However, with the intraocular lens 10 of structure according to this example, the projecting parts 32, 32 are formed on the distal end portions of the supporting portions 16, 16 which will be superposed against the optical surface of the optical portion 12. By means of this design, problems such as failure of the supporting portions 16, 16 to deploy within the eye due to adhesion of the supporting portions 16, 16 to the optical portion 12 will occur with difficulty, even where the intraocular lens 10 employs an acrylic polymer exhibiting viscosity. Specifically, when the supporting portion posterior surfaces 28, 28 are superposed against the optical portion anterior surface 18, the projecting parts 32, 32 which project out toward the posterior surface side at the distal edges of the supporting portions 16, 16 will come into contact with the optical portion anterior surface 18, whereby the optical portion 12 and the supporting portions 16, 16 will come into partial contact with a much smaller contact area as compared to the case where the supporting portion posterior surfaces 28, 28 are positioned in contact in their entirety against the optical portion anterior surface 18. Moreover, since the projecting parts 32, 32 in this example in particular have generally semispherical shape, the projecting distal ends of the projecting parts 32, 32 assume a state of point contact against the optical portion anterior surface 18, whereby a small contact area can be provided even more advantageously. Thus, with the intraocular lens 10 pertaining to this example, despite the fact that the supporting portions 16, 16 are folded onto the optical portion 12 during insertion, adhesion force acting between the supporting portions 16, 16 and the optical portion 12 will be sufficiently low to make adhesion difficult, so that even if temporary adhesion does occur, once inserted into the eye the supporting portions 16, 16 will easily disadhere through resilient force and will be able to recover their initial shape.

Moreover, with the intraocular lens 10 in this example, recovery of the intraocular lens to its initial shape within the eye can be accomplished advantageously without any special procedure; and once the intraocular lens 10 has deployed within the eye, through positional adjustment of the intraocular lens 10 within the capsule using an appropriate instrument as needed, the intraocular lens 10 can be positioned with respect to the capsule in such a way that the optical axis of the optical portion 12 is generally aligned with the ophthalmic center axis.

In the one-piece intraocular lens 10 of structure according to the present embodiment, the projecting parts 32, 32 are formed so as to project out from the supporting portion posterior surfaces 28, 28 at the projecting distal end portions of the supporting portions 16, 16. By means of this design, when the supporting portions are superposed against and placed in contact with the optical surface of the optical portion 12 (the optical portion anterior surface 18) during insertion of the intraocular lens 10 into the eye, contact of the projecting distal ends of the projecting parts 32, 32 will prevent the supporting portion posterior surfaces 28, 28 from coming into contact over their entire face against the optical surface of the optical portion 12. For this reason, the area of adhesion can be kept smaller as compared to the case where the supporting portion posterior surfaces 28, 28 come into contact over their entire face against the optical surface of the optical portion 12, thus preventing a high level of adhesive force from developing. Consequently, despite the relatively low resilient force of the supporting portions 16, 16 of narrow, elongated rod shape, they will disadhere to a sufficient extent for the supporting portions 16, 16 to deploy through resilient force after insertion of the intraocular lens 10 into the eye, so as to consistently recover their initial shape.

In this example, the projecting parts 32, 32 are given semispherical shape, whereby the projecting distal ends of the projecting parts 32, 32 come into point contact against the optical portion 12. For this reason, the area of contact of the supporting portions 16, 16 (the projecting parts 32, 32) against the optical surface can be more advantageously reduced in size, and the effect of lowering adhesive force can be effectively achieved.

Furthermore, the projecting parts 32, 32 are formed so as to project towards the posterior surface side from the projecting distal end portions of the supporting portion posterior surfaces 28, 28, whereas the supporting portion anterior surfaces 26, 26 are constituted as flat surfaces. By so doing, the projecting parts 32, 32 are formed on the supporting portion posterior surfaces 28, 28 which will be superposed against the optical portion posterior surfaces 20, 20 so that the area of contact between the optical portion posterior surfaces 20, 20 and the supporting portion posterior surfaces 28, 28 can be made sufficiently small; and the supporting portion anterior surfaces 26, 26 will be constituted as flat surfaces, thereby preventing the thickness dimension of the supporting portions 16, 16 from being larger than necessary, so that the intraocular lens 10 can be folded or rolled to sufficiently small size.

In the following examples, parts and areas substantially identical to those of the intraocular lens 10 in the preceding example will be assigned the same symbols in the drawings as in the first example, and will not be discussed in any detail.

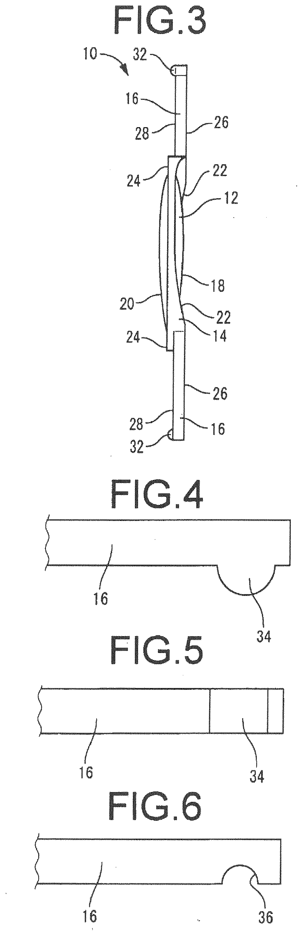

FIGS. 4 and 5 depict the relevant portion of an intraocular lens. In the intraocular lens a single rib part 34 is formed as a viscosity reducing portion, on the supporting portion posterior surface 28 of the pair of supporting portions 16, 16. This rib part 34 extends continuously with a generally unchanging semicircular cross section in the width direction of the supporting portions 16, 16, and is constituted so as to have a bowing surface which is convex towards the side to which the rib part 34 projects.

The intraocular lens of structure affords effects similar to those of the previous example in that it effectively prevents adhesion of the supporting portions 16, 16 to the optical portion 12, and enables consistent recovery of the supporting portions 16, 16 to their original shape after insertion into the eye. Moreover, with the intraocular lens in the folded or rolled state for insertion into the eye, the supporting portions 16, 16 and the optical portion 12 will be in linear contact. Thus, as compared to the first embodiment, it will be possible to advantageously reduce or avoid contact between the supporting portions 16, 16 and the optical portion 12 in portions other than the rib part 34, and to achieve more consistent viscosity reducing effect.

FIGS. 6 and 7 depict the relevant portion of an intraocular lens. Specifically, in the intraocular lens pertaining to the present embodiment, a single recess 36 that opens onto the posterior face side of the supporting portions 16, 16 is formed by way of a viscosity reducing portion. The recess 36 is formed so as to extend through the projecting distal end portion of the supporting portions 16, 16, with generally unchanging semicircular cross section in the width direction of the supporting portions 16, 16 and with the widthwise ends of the recess opening onto the widthwise side edges of the supporting portions 16, 16.

The intraocular lens of structure affords reduced contact area of the projecting distal end portions of the supporting portions 16, 16, which have the problem of being particularly prone to adhesion, vis-à-vis other areas (the optical surface of the optical portion 12 and so on), and can thus reduce adhesive force. Moreover, the adhesive force reducing action afforded by the recess 36 can effectively prevent increased thickness in the optical axis direction in the projecting distal end portions of the supporting portions 16, 16, thus more advantageously achieving compact size of the intraocular lens in the folded or rolled state for insertion into the eye.

Next, FIG. 8 depicts the relevant portion of an intraocular lens pertaining to an embodiment in accordance with the present invention. Specifically, in the intraocular lens pertaining to the present embodiment, the viscosity reducing portions are constituted by rough surface portions 38, 38 created by a surface roughening process to form very large numbers of tiny irregularities (e.g. irregularities of ≤ 1 mm width and height) on the supporting portion posterior surfaces 28, 28 of the supporting portions 16, 16. The rough surface portions 38, 38 in the present embodiment are composed of a multitude tiny grooves resembling scratches formed in a generally lattice pattern; the surface roughening process can be advantageously accomplished by subjecting the surface of the forming mold used in the molding process to a treatment such as shotblasting or etching, or to laser irradiation, to form the irregularities.

The intraocular lens of structure according to the present embodiment, effectively affords viscosity reducing effect of the supporting portions 16, 16 with respect to the optical portion 12, as well as minimizing the thickness dimension at the projecting distal end portions of the supporting portions 16, 16 so as to advantageously achieve compact size of the intraocular lens during insertion into the eye.

Moreover, in the intraocular lens of the present embodiment, the rough surface portions 38, 38 formed on the supporting portions 16, 16 are produced by a surface roughening process, thereby reducing transparency of the supporting portions 16, 16 at the locations where the rough surface portions 38, 38 have been formed and improving their visibility in liquid. Specifically, in the past it has been attempted to improve visibility of the supporting portions in liquids with a view to improving manipulability of the intraocular lens within the eye, an operation which is carried out substantially within liquid; and to this end, means such as imparting color to the supporting portions to the supporting portions have been proposed. However, such methods had the problems of being difficult to implement in intraocular lenses of one-piece type, or of imposing unavoidable limitations as to materials. In the intraocular lens of the present embodiment, in order to improve manipulability of the intraocular lens within the eye, an operation which is carried out substantially within liquid, the rough surface portions 38, 38 are formed on the supporting portions 16, 16, thereby reducing the transparency of the supporting portions 16, 16 and successfully ensuring adequate visibility within liquids. By so doing, when the intraocular lens is positioned using a specific instrument after being inserted into the eye, visibility of the supporting portions 16, 16 within the eye filled aqueous humor, viscoelastic substance, or the like will be enhanced, and manipulability of the intraocular lens within the eye can be improved.

The shape of the projections formed on the supporting portions is not limited in any way to the specific disclosure regarding the projecting parts 32 in the preceding first embodiment. Specifically, FIGS. 9 and 10 depict the relevant portion of the distal end portion of the supporting portions 16, 16 of an intraocular lens. Specifically, the supporting portions 16, 16 depicted in FIGS. 9 and 10 have projecting parts 40, 40 of three-sided pyramid shape. The intraocular lens having projecting parts 40, 40 of such shape affords effects similar to the preceding first example.

FIGS. 11 and 12 depict the relevant portion of the projecting distal end portion of the supporting portions 16, 16 of an intraocular lens. The projecting distal end portions of the supporting portions 16, 16 of the intraocular lens have formed thereon projecting parts 42, 42 of generally rectangular parallelepiped shape which project out in the direction of the optical axis. In the intraocular lens furnished with projecting parts 42, 42 of this shape, the contact area thereof with the optical portion 12 will be greater than with the projecting parts 42, 42 shown in the preceding first example. Thus, a more stable condition of contact can be achieved; portions of the supporting portions 16, 16 other than those where the projecting parts 42, 42 can be effectively prevented from contacting the optical portion 12; and adhesion of the supporting portions 16, 16 to the optical portion 12 can be effectively reduced.

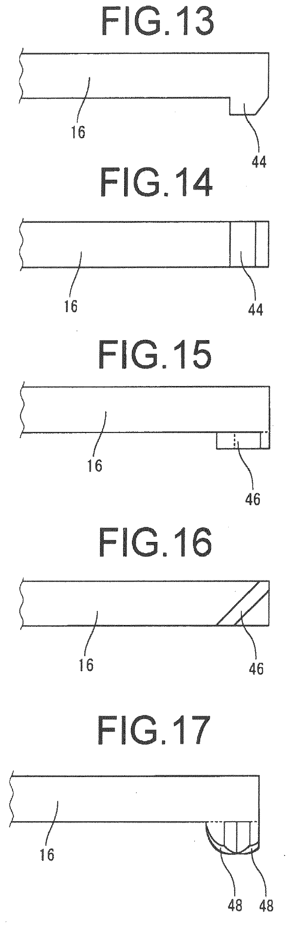

FIGS. 13 and 14 depict the relevant portion of the projecting distal end portion of the supporting portions 16, 16 of an intraocular lens. The projecting distal end portions of the supporting portions 16, 16 of the intraocular lens pertaining to the present embodiment have formed thereon projecting parts 44, 44 which are formed so as to project out in the direction of the optical axis and extend in the width direction with a generally unchanging trapezoidal cross section. In the present embodiment in particular, the anterior edges of the supporting portions 16, 16 at the projecting distal end portions of the projecting parts 44, 44 are constituted by an obtuse angle. The intraocular lens furnished with projecting parts 44, 44 of this shape affords effects substantially identical to those of the preceding example, as well as being able in particular to ameliorate irritation caused by contact with the capsule, owing to the obtuse angle of the projecting distal end portions of the supporting portions 16, 16 which in most instances will be disposed in firm contact against the inside surface of the capsule.

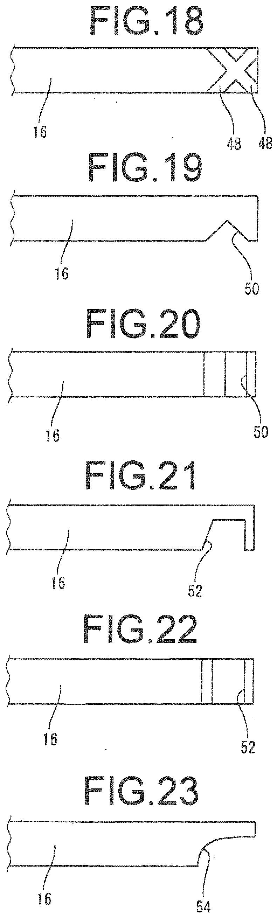

Moreover, the shape of the ribs is not limited in any way by the specific disclosure regarding the rib parts 34, 34 in the preceding second example, and it would be acceptable to employ rib parts having any of various cross sectional shapes, such as a quadrilateral shape or triangular shape. Furthermore, as with the rib parts 46, 46 formed on the supporting portions 16, 16 of an intraocular lens pertaining to an eighth embodiment depicted in FIGS. 15 and 16, the rib portions may be formed so as to extend diagonally in the direction perpendicular to the optical axis, with respect to the width direction of the supporting portions. Moreover, the cross sectional shape of the rib parts need not necessarily be unchanging; as with the rib parts 48, 48 formed on the supporting portions 16, 16 of an intraocular lens pertaining to another example depicted in FIGS. 17 and 18, the rib parts may have variable projecting height in the direction of their extension. The rib parts 48, 48 are formed by two ribs intersecting at approximately right angles in rear view.

The recesses formed in the supporting portions 16, 16 are not limited in any way by the specific disclosure regarding the recesses 36, 36 in the preceding third example. As a specific example, FIGS. 19 and 20 depict the relevant portion of the projecting distal end portion of the supporting portions 16, 16 of an intraocular lens pertaining to a tenth embodiment. The projecting distal end portions of the supporting portions 16, 16 have formed therein recesses 50, 50 that extend with generally unchanging triangular cross section along the width direction of the supporting portions 16, 16 so as to open onto either widthwise side face of the supporting portions 16, 16. FIGS. 21 and 22 depict the relevant portion of the projecting distal end portion of the supporting portions 16, 16 of an intraocular lens. The projecting distal end portions of the supporting portions 16, 16 have formed therein recesses 52, 52 that extend with generally unchanging trapezoidal cross section so as to open onto either widthwise side face of the supporting portions 16, 16. The intraocular lenses of structure according to the tenth and eleventh embodiments afford effects similar to those of the intraocular lens in the third embodiment. The recess constituting the viscosity reducing portion need not necessary have unchanging cross section, and may be formed with variable recess depth, recess width, and so on.

Moreover, the recesses may be formed so as to open to either side in the width direction, or to open on the projecting distal end. By way of a specific example, as shown in FIGS. 23 and 24, by means of forming a notch part 54 such that the posterior surface side of the projecting distal end of the supporting portions 16, 16 extends along the width direction with a generally quarter-circular shape, it is possible to reduce the contact area of the distal ends of the supporting portions 16, 16 against the optical portion 12 when the intraocular lens is folded or rolled up, thus affording viscosity reducing effect.

Furthermore, where the supporting portions 16, 16 are furnished with rough surface portions are furnished by way of the viscosity reducing portions as taught in the preceding fourth example, the rough surface portions are not limited to rough surface portions 38, 38 composed of grooves in a diagonal lattice pattern as shown in the preceding fourth example, and may instead employ various other morphologies such as a multitude of microscopic irregularities, grooves, or the like. By way of a specific example, FIG. 25 depicts rough surface portions 56, 56 formed on the projecting distal end portions of the supporting portions 16, 16 of an intraocular lens pertaining to another embodiment in accordance with the present invention. Specifically, the rough surface portions 56, 56 in the present embodiment have a grained pattern formed so as to extend generally in the width direction. That is, the rough surface portion 56 has a multitude of fine linear irregularities extending generally in the width direction of the supporting portion 16, producing an overall irregularity pattern that resembles a bar code. The intraocular lens of structure according to the present embodiment furnished with such rough surface portions 56, 56 affords effects substantially identical to those of the preceding fourth embodiment.

Moreover, the aforementioned projections, ribs, recesses, and rough surface portions may all be formed in plural number at multiple locations on the supporting portions 16, 16. As a specific example, the viscosity reducing portions could be constituted with morphologies such as those depicted in FIGS. 26 through 31. By forming a plurality of projections, ribs, recesses, or rough surface portions in the supporting portion 16 as shown in FIGS. 26 through 31, the condition of contact of the supporting portions 16, 16 against the optical portion can be stabilized, and the effect of reduced adhesion of the supporting portions 16, 16 to the optical portion 12 can be achieved.

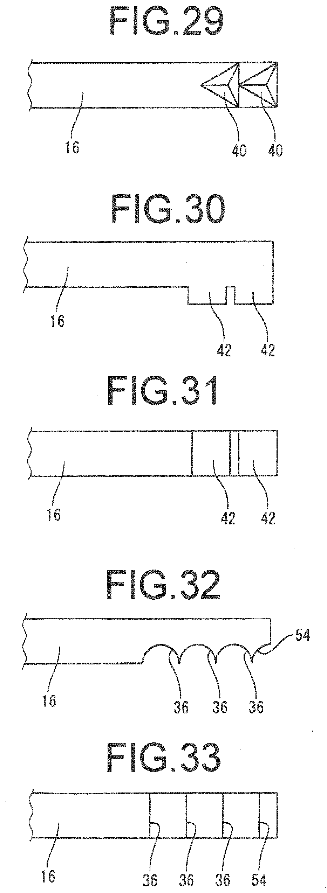

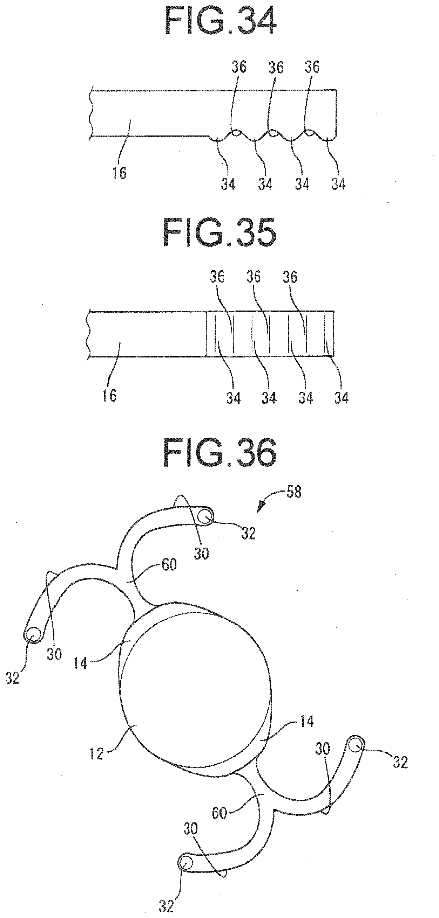

Furthermore, it is possible for the projecting parts 32, 40, 42, 44, the rib parts 34, 46, 48, the recesses 36, 50, 52, or the rough surface portions 38, 56 to be formed in combination in plural number on the supporting portions 16, 16. As a specific example, in the relevant portion of an intraocular lens shown in FIGS. 32 and 33, a plurality of recesses 36, 36, 36 are formed on the supporting portions 16, 16, while a notch part 54 is formed in the projecting distal end portion. In the relevant portion of the intraocular lens shown in fragmentary view in FIGS. 32 and 33, thin ribs that extend in the width direction are formed between adjacent recesses 36, 36 and between the recess 36 and the notch part 54; contact of these ribs against the optical portion 12 serves to minimize the contact surface area between the supporting portions 16, 16 and the optical portion 12 to achieve adhesion reducing effect. In particular, several of these ribs can be produced by means of forming a plurality of recesses 36, 36, 36 and a notch part 54, to achieve a condition of stable contact thereby. On the supporting portions 16, 16 of the intraocular lens depicted in FIGS. 34 and 35, a plurality of rib parts 34, 34, 34, 34 have been formed, with a single recess 36 being formed between each two adjacent ribs. With an intraocular lens like that depicted in FIGS. 34 and 35, satisfactory projecting height of the rib parts 34, 34, 34, 34 an be achieved without making the supporting portions 16, 16 excessively thick at locations where the viscosity reducing portions have been formed; and consistent adhesion reducing effect can be achieved. Moreover, through appropriate combination of a plurality of projections, ribs, grooves, and/or rough surfaces, excellent effects can be advantageously achieved through the combined effects of various morphologies formed on the supporting portion 16.

Moreover, it is not necessary for the recesses or rough surface portions to be formed on only one surface selected from the anterior and posterior surfaces of the supporting portions 16, 16, and it would be acceptable, for example, to form recesses etc. on both the anterior and posterior surfaces 26, 28 of the supporting portions. By so doing, adhesion reducing effect can be effectively achieved regardless of whether the anterior or the posterior surface of the supporting portions 16, 16 becomes superposed against the optical portion 12. Formation of viscosity reducing portions on both surfaces of the supporting portions in this manner is particularly effective in the supporting portions 16, 16 that will be positioned at the leading end in the direction of insertion during insertion of the intraocular lens into the eye. Specifically, since the supporting portions 16, 16 situated on the leading end in the direction of insertion will naturally become bent backward and superposed against the optical portion 12 due to friction etc. with the peripheral wall of the insertion instrument as the lens is being pushed by the plunger, it is relatively difficult to bring about folding so as to become superposed on a specific surface of the optical portion 12. Thus, the effect of the providing both the anterior and posterior surfaces 26, 28 with viscosity reducing portions may be effectively achieved. Since formation of rough surface portions on both the anterior and posterior supporting portion surfaces 26, 28 will not readily give rise to problems such as localized thinning or thickening of the supporting portions 16, 16, rough surface portions are particularly favorably employed in cases where it is desired to form viscosity reducing portions on both the anterior and posterior surfaces 26, 28 of the supporting portions 16, 16.

In preferred practice, the projecting parts 32, 40, 42, 44, the rib parts 34, 46, 48, the recesses 36, 50, 52, or the rough surface portions 38, 56 will be formed so as to include the projecting distal end portion of the supporting portions 16, 16 which are particularly prone to becoming superposed against the optical portion 12 with the lens in the folded or rolled state during insertion into the eye; however, the location for forming the projecting parts, rib parts, recesses rough surface portions, and so on is not limited to the projecting distal end portion of the supporting portions 16, 16, and may instead be formed in the medial section lying in the direction of projection of the supporting portions 16, 16, or formed extending across generally the entire face of the supporting portions 16, 16 from the end on the basal end side to the projecting distal end.

The shape of the supporting portions 16, 16 is in no way limited by the specific disclosures in the preceding embodiments. As specific examples, supporting portions of various shapes such as those shown in FIGS. 36 to 39 are possible.

Specifically, in the intraocular lens 58 depicted in FIG. 36, the supporting portions 60, 60 branch in their medial section into two forks, with portions lying further towards the projecting distal end from the medial section respectively bowing along the circumferential direction of the optical portion 12 and extending in mutually opposite directions. The intraocular lens 58 having the supporting portions 60, 60 of this design affords greater stability of and more secure support the optical portion 12 when inserted into the capsule.

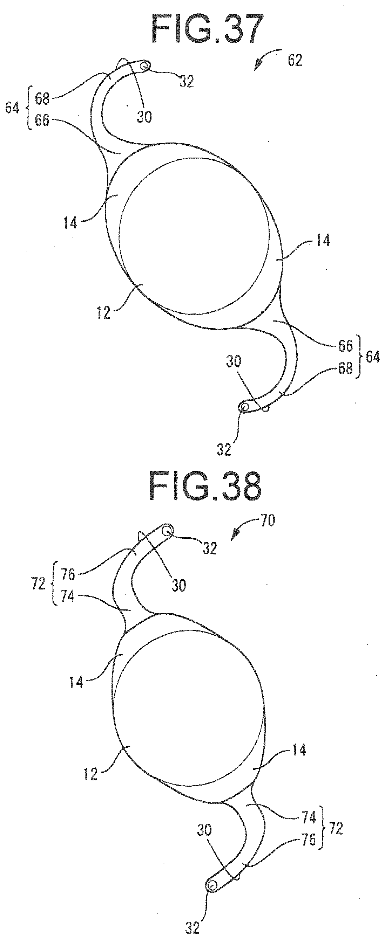

In another intraocular lens 62 depicted in FIG. 37, the basal end portion 66 of the supporting portions 64, 64, which is the side thereof that connects to the outer circumferential surface of the coupling portion 14, is wider in shape than it distal end portion 68; and the distal end portion 68 is bowed along the circumferential direction of the optical portion 12. The intraocular lens 62 having the supporting portions 64, 64 of this design can ensure sufficient rigidity by means of the relatively wide basal end portion 66, while affording stable positioning and immobilization of the optical portion 12 at the prescribed location, and affording a sufficient level of pliability owing to the relatively thin distal end portion 68, thereby preventing injury to capsule due to the supporting portions 64, 64 coming into contact with the inside surface of the capsule, as well as avoiding damage to the intraocular lens 62 by contact reaction force.

In yet another intraocular lens 70 depicted in FIG. 38, the supporting portion 72 extends in a generally linear rod configuration, as well as bending in the optical axis-perpendicular direction in its medial section, while the basal end portion 74 situated towards the coupling portion 14 from this medial section is wider in shape relative to its projecting distal end portion 76. The intraocular lens 70 having the supporting portions 72, 72 of this design affords effects similar to the intraocular lens 62 shown in the preceding FIG. 37, as well as affording stronger mating of the projecting distal ends of the supporting portions 72, 72 against the inside surface of the capsule.

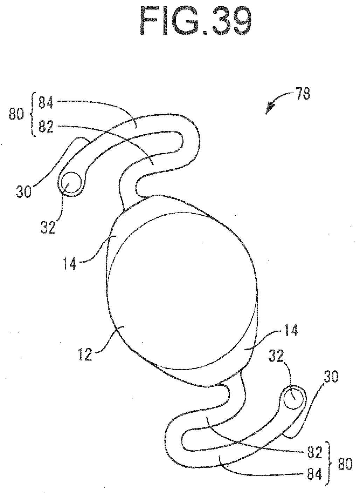

In yet another intraocular lens 78 depicted in FIG. 39, the supporting portion 80 as a whole is bent into a generally hooked configuration. In more detail, the pair of supporting portions 80, 80 are situated in opposition along one direction across the diameter of the optical portion 12 and project outwardly in the diametrical direction with the supporting portions bent or bowed in the medial section to form a pair of medial portions 82, 82 that extend a given length along the circumferential direction of the optical portion 12 and that extend in mutually opposite directions in the circumferential direction. These medial portions 82, 82 are in turn bent or bowed in sections thereof to form distal end portions 84, 84 extending in mutually opposite directions in the circumferential direction with respect to the medial portions 82, 82. In the present embodiment, the medial portions 82 and the distal end portions 84 extend in a generally concentric circular arrangement, with the distal end portions 84 having greater radius of curvature than the medial portions 82. The intraocular lens 78 having the supporting portions 80 of this design, by virtue of the hook configuration produced by a hairpin bend, can advantageously cushion stress acting on the capsule due to contact when the intraocular lens in implanted inside the capsule, thus avoiding the problem of injury to the capsule.

Furthermore, as with the supporting portions 64, 72 depicted in FIGS. 37 and 38, the width dimension of the supporting portions may vary from the basal end to the distal end; and the thickness dimension, i.e. the dimension of the supporting portions in the direction of the optical axis, may be established appropriately depending on properties such as the strength required of the supporting portions. As a specific example, the supporting portion may become gradually thinner towards its projecting distal end; where such a supporting portion is employed, strength in the projecting distal end portion will be lower, and reaction force during contact can be reduced while at the same time ensuring adequate strength at the basal end portion, so as to achieve adequate ability of the supporting portion to position the intraocular lens.

In preferred practice, in order to reduce concentration of stress at the zone of connection between the supporting portion and the coupling portion, a widened part of rounded shape will be provided to either widthwise side of the end of the supporting portion on the basal end side thereof, to provide a gradual increase in width towards the portion of connection to the coupling portion. However, it will not always be necessary to provide such a widened part of rounded shape at the end of the supporting portion on its basal end side.

The coupling portions 14, 14 will not always be necessary, and the pair of supporting portions 16, 16 may instead be formed so as to extend directly outward in the optical axis-perpendicular direction from the outer fringe part of the optical portion 12. The shape of the coupling portions is in no way limited by the specific disclosures in the preceding embodiments, and various shapes may be employed appropriately for it, such as a generally annular shape formed about the entire circumference, or the like.

## Claims

1. An intraocular lens (10, 58, 62, 70, 78) of one-piece type comprising:
an optical portion (12) of generally circular shape in front view and including a lens zone having prescribed optical characteristics; and
supporting portions (16, 60, 64, 72, 80) that extend radially outwardly from the optical portion (12), and that with the lens (10, 58, 62, 70, 78) inserted in an eye, are configured to be disposed in contact against an inside surface of an outer circumferential part of a capsule thereby holding the optical portion (12) positioned within the capsule, each supporting portion (16, 60, 64, 72, 80) having a projecting distal end side and a basal end side, the optical portion (12) and supporting portions (16, 60, 64, 72, 80) are integrally formed with a foldable or rollable soft material, **characterized in that**
a viscous material is employed as the soft material,
the supporting portions (16, 60, 64, 72, 80) have rough surface portions (38,56) with reduced viscosity at least at portions that are adapted to be superposed on the optical portion (12) when the lens (10, 58, 62, 70, 78) is folded or rolled and inserted in to the eye,
the supporting portions (16, 60, 64, 72, 80) have a reduced transparency by means of the rough surface portions (38, 56) in comparison with the optical portion (12), and
the rough surface portions (38,56) are formed at least on the projecting distal end sides and basal end sides of the supporting portions (16, 60, 64, 72, 80).

2. The intraocular lens according to claim 1, wherein the rough surface portions (38, 56) are formed on both of an anterior surface (26) and a posterior surface (28) of the supporting portions (16, 60, 64, 72, 80).

3. The intraocular lens according to claim 1, wherein the rough surface portions (38, 56) are formed on either an anterior surface (26) or a posterior surface (28) of the supporting portions (16, 60, 64, 72, 80).

4. The intraocular lens according to any one of claims 1-3, wherein the rough surface portions (38, 56) comprise a very large number of tiny irregularities.

5. The intraocular lens according to any one of claims 1-3, wherein the rough surface portions (56) comprise a multitude of fine linear recesses extending in a width direction of the supporting portions (16, 60, 64, 72, 80).

6. The intraocular lens according to any one of claims 1-5, wherein the rough surface portions (38, 56) of the supporting portions (16, 60, 64, 72, 80) are visible in a liquid.

7. The intraocular lens according to any one of claims 1-6, wherein the rough surface portions (38, 56) are formed from basal end sides to projecting distal ends of the supporting portions (16, 60, 64, 72, 80).

8. The intraocular lens according to any one of claims 1-7, wherein each of the supporting portions (16, 60, 64, 72, 80) has a rectangular cross section with a flat anterior surface (26) and a flat posterior surface (28), and the rough surface portions (38, 56) are formed on either the anterior surfaces (26) or the posterior surfaces (28) of the supporting portions (16, 60, 64, 72, 80).

9. The intraocular lens according to any one of claims 1-8, wherein the intraocular lens is a lens for treating a cataract, and is configured to be inserted into a capsule from which a lens of the eye has been removed.

10. The intraocular lens according to any one of claims 1-9, wherein the supporting portions (16, 60, 64, 72, 80) comprises a pair of supporting portions (16, 60, 64, 72, 80) projecting out to either side in an insertion direction, and the intraocular lens is configured to be inserted into the eye by means of an insertion instrument with the pair of supporting portions (16, 60, 64, 72, 80) both folded and superposed on the optical portion (12).

## Patentansprüche

1. Intraokularlinse (10, 58, 62, 70, 78) der einteiligen Bauart, enthaltend:
einen optischen Teil (12) mit allgemein kreisförmiger Form in der Vorderansicht, der eine Linsenzone einschließt, die vorgeschriebene optische Eigenschaften hat; und
Halteteile (16, 60, 64, 72, 80), die sich von dem optischen Teil (12) radial nach außen erstrecken und die bei der in ein Auge eingesetzten Linse (10, 58, 62, 70, 78) so konfiguriert sind, dass sie in Kontakt mit einer inneren Oberfläche eines äußeren Umfangsteils einer Kapsel angeordnet werden und dadurch den optischen Teil (12) innerhalb der Kapsel positioniert halten, wobei jeder Halteteil (16, 60, 64, 72, 80) eine vorspringende distale Endseite und eine Basisendseite hat, der optische Teil (12) und die Halteteile (16, 60, 64, 72, 80) einstückig mit einem faltbaren oder rollbaren weichen Material geformt sind, **dadurch gekennzeichnet, dass**
ein viskoses Material als das weiche Material verwendet wird,
die Halteteile (16, 60, 64, 72, 80) raue Oberflächenteile (38, 56) mit einer reduzierten Viskosität zumindest in Teilen haben, die dafür ausgelegt sind, über den optischen Teil (12) gelegt zu werden, wenn die Linse (10, 58, 62, 70, 78) gefaltet oder gerollt und in das Auge eingesetzt wird,
die Halteteile (16, 60, 64, 72, 80) im Vergleich zu dem optischen Teil (12) eine reduzierte Transparenz mittels der rauen Oberflächenteile (38, 56) haben, und
die rauen Oberflächenteile (38, 56) zumindest an den vorspringenden distalen Endseiten und den Basisendseiten der Halteteile (16, 60, 64, 72, 80) gebildet sind.

2. Intraokularlinse nach Anspruch 1, wobei die rauen Oberflächenteile (38, 56) sowohl auf einer vorderen Oberfläche (26) als auch einer rückseitigen Oberfläche (28) der Halteteile (16, 60, 64, 72, 80 gebildet sind.

3. Intraokularlinse nach Anspruch 1, wobei die rauen Oberflächenteile (38, 56) entweder auf einer vorderen Oberfläche (26) oder einer rückseitigen Oberfläche (28) der Halteteile (16, 60, 64, 72, 80) gebildet sind.

4. Intraokularlinse nach einem der Ansprüche 1-3, wobei die rauen Oberflächenteile (38, 56) eine sehr große Anzahl von winzigen Unregelmäßigkeiten umfassen.

5. Intraokularlinse nach einem der Ansprüche 1-3, wobei die rauen Oberflächenteile (56) eine Vielzahl von feinen linearen Vertiefungen umfassen, die in einer Breitenrichtung der Halteteile (16, 60, 64, 72, 80) verlaufen.

6. Intraokularlinse nach einem der Ansprüche 1-5, wobei die rauen Oberflächenteile (38, 56) der Halteteile (16, 60, 64, 72, 80) in einer Flüssigkeit sichtbar sind.

7. Intraokularlinse nach einem der Ansprüche 1-6, wobei die rauen Oberflächenteile (38, 56) von den Basisendseiten zu den vorspringenden distalen Enden der der Halteteile (16, 60, 64, 72, 80) gebildet sind.

8. Intraokularlinse nach einem der Ansprüche 1-7, wobei die Halteteile (16, 60, 64, 72, 80) jeweils einen rechteckigen Querschnitt mit einer flachen vorderen Oberfläche (26) und einer flachen rückseitigen Oberfläche (28) haben und die rauen Oberflächenteile (38, 56) entweder auf den vorderen Oberflächen (26) oder den rückseitigen Oberflächen (28) der Halteteile (16, 60, 64, 72, 80) gebildet sind.

9. Intraokularlinse nach einem der Ansprüche 1-8, wobei die Intraokularlinse eine Linse zur Behandlung eines Katarakts ist und dafür konfiguriert ist, dass sie in eine Kapsel eingesetzt wird, aus welcher eine Linse des Auges entfernt wurde.

10. Intraokularlinse nach einem der Ansprüche 1-9, wobei die Halteteile (16, 60, 64, 72, 80) ein Paar von Halteteilen (16, 60, 64, 72, 80) umfassen, die in einer Einführrichtung an beiden Seiten nach außen vorspringen, und die Intraokularlinse dafür konfiguriert ist, in das Auge mittels eines Einsetzinstruments eingesetzt zu werden, wobei beide Halteteile (16, 60, 64, 72, 80) des Paares gefaltet und auf den optischen Teil (12) gelegt sind.

## Revendications

1. Lentille intraoculaire (10, 58, 62, 70, 78) de type monobloc comprenant :
une partie optique (12) de forme généralement circulaire en vue frontale et incluant une zone de lentille présentant des caractéristiques optiques prescrites ; et
des parties de support (16, 60, 64, 72, 80) qui s'étendent radialement vers l'extérieur à partir de la partie optique (12), et qui avec la lentille (10, 58, 62, 70, 78) insérée dans un oeil, sont configurées pour être placées en contact contre une surface intérieure d'une partie circonférentielle extérieure d'une capsule maintenant ainsi la partie optique (12) positionnée à l'intérieur de la capsule, chaque partie de support (16, 60, 64, 72, 80) présentant un côté d'extrémité distale faisant saillie et un côté d'extrémité basale, la partie optique (12) et les parties de support (16, 60, 64, 72, 80) sont formées intégralement d'une matière souple pliable ou roulable, **caractérisée en ce que**
une matière visqueuse est employée en tant que la matière souple ;
les parties de support (16, 60, 64, 72, 80) présentent des parties de surfaces rugueuses (38, 56) de viscosité réduite au moins au niveau de parties qui sont adaptées à être superposées sur la partie optique (12) lorsque la lentille (10, 58, 62, 70, 78) est pliée ou roulée, et insérée dans l'oeil ;
les parties de support (16, 60, 64, 72, 80) ont une transparence réduite au moyen des parties de surfaces rugueuses (38, 56) par rapport à la partie optique (12), et
les parties de surfaces rugueuses (38, 56) sont formées au moins sur les côtés d'extrémité distale faisant saillie et les côtés d'extrémité basale des parties de support (16, 60, 64, 72, 80).

2. Lentille intraoculaire selon la revendication 1, dans laquelle les parties de surfaces rugueuses (38, 56) sont formées à la fois sur une surface antérieure (26) et sur une surface postérieure (28) des parties de support (16, 60, 64, 72, 80).

3. Lentille intraoculaire selon la revendication 1, dans laquelle les parties de surfaces rugueuses (38, 56) sont formées sur une surface antérieure (26) ou sur une surface postérieure (28) des parties de support (16, 60, 64, 72, 80) .

4. Lentille intraoculaire selon l'une quelconque des revendications 1 à 3, dans laquelle les parties de surfaces rugueuses (38, 56) comportent un très grand nombre de petites irrégularités.

5. Lentille intraoculaire selon l'une quelconque des revendications 1 à 3, dans laquelle les parties de surfaces rugueuses (56) comportent une multitude d'évidements linéaires fins s'étendant dans une direction de largeur des parties de support (16, 60, 64, 72, 80).

6. Lentille intraoculaire selon l'une quelconque des revendications 1 à 5, dans laquelle les parties de surfaces rugueuses (38, 56) des parties de support (16, 60, 64, 72, 80) sont visibles dans un liquide.

7. Lentille intraoculaire selon l'une quelconque des revendications 1 à 6, dans laquelle les parties de surfaces rugueuses (38, 56) sont formées depuis des côtés d'extrémité basale jusqu'à des extrémités distales faisant saillie à partir des parties de support (16, 60, 64, 72, 80) .

8. Lentille intraoculaire selon l'une quelconque des revendications 1 à 7, dans laquelle chacune des parties de support (16, 60, 64, 72, 80) présente une section transverse rectangulaire avec une surface antérieure plate (26) et une surface postérieure plate (28), et les parties de surfaces rugueuses (38, 56) sont formées sur les surfaces antérieures (26) ou sur les surfaces postérieures (28) des parties de support (16, 60, 64, 72, 80).

9. Lentille intraoculaire selon l'une quelconque des revendications 1 à 8, dans laquelle la lentille intraoculaire est une lentille destinée au traitement de la cataracte, et est configurée de manière à être insérée dans une capsule de laquelle une lentille de l'oeil a été retirée.

10. Lentille intraoculaire selon l'une quelconque des revendications 1 à 9, dans laquelle les parties de support (16, 60, 64, 72, 80) comportent une paire de parties de support (16, 60, 64, 72, 80) faisant saillie vers chaque côté dans une direction d'insertion, et dans laquelle la lentille intraoculaire est configurée de manière à être insérée dans l'oeil au moyen d'un instrument d'insertion avec la paire de parties de support (16, 64, 72, 80, 60) repliée et superposée sur la partie optique (12).
